# EUROPEAN PATENT SPECIFICATION

(11) **EP 1 441 652 B1**
(45) Date of publication and mention of the grant of the patent: **31.08.2005**
(21) Application number: 02735821.7
(22) Date of filing: 10.06.2002
(51) Int. Cl.: A61B 17/16, F16D 1/064

(54) **TORQUE-TRANSMITTING COUPLING AND SURGICAL DEVICE**
DREHMOMENTÜBERTRAGENDE KUPPLUNG UND CHIRURGISCHE VORRICHTUNG
COUPLAGE DE TRANSMISSION DE COUPLE ET DISPOSITIF CHIRURGICAL

(30) Priority: 05.11.2001 US 338718 P; 10.01.2002 US 43423; 22.01.2002 US 55806; 31.01.2002 US 59232
(43) Date of publication of application: 04.08.2004
(73) Proprietor: PRECIMED S.A., 2534 Orvin (CH)
(72) Inventor: LECHOT, André, CH-2534 Orvin (CH)
(74) Representative: Grosfillier, Philippe
(86) International application number: PCT/IB2002/002219
(87) International publication number: WO 2003/039380

(56) References cited:
- EP-A- 0 682 917
- US-A- 5 499 984
- US-A- 5 693 047
- PATENT ABSTRACTS OF JAPAN vol. 1996, no. 02, 29 February 1996 (1996-02-29) & JP 07 265326 A (OLYMPUS OPTICAL CO LTD), 17 October 1995 (1995-10-17)

## Description

### Technical Field

This invention generally relates to torque-transmitting coupling assemblies useful with flexible shafts made of super-elastic alloy, which are particularly beneficial in surgical instrumentation, e.g., flexible reaming systems.

### Background Art

A torque transmitting assembly according to the preamble of claim 1 is known from US-A-5,499,984.

Torque has been traditionally transmitted across non-concentric axes via universal joints. Some have used drive shafts of a coiled spring construction that bend while transmitting torque along a desired path, to ameliorate the surrounding impact of protrusions from such joints. These connections have proven undesirable, particularly in surgical operations. For blood and other organic matter trapped in the assembly cannot easily be cleaned due to the intricate, non-exposed surfaces that are largely inaccessible. Moreover, aspects of universal joints can move against the tissue of a patient to cause a deleterious effect.

One approach has been to utilize flexible tubular shafts made of a super-elastic alloy such as nickel-titanium (e.g., Nitinol) to transmit torque, which has met with some success. Using a tube to transmit torque generally minimizes unexposed surfaces and allows easier cleaning, as there are no surfaces extending beyond the torque transmission tube. Universal joints instead protrude beyond the envelope of the drive shaft. Thus, the use of elastic tubes made of super-elastic alloy in, e.g., surgical devices substantially reduces the possibility of harm or discomfort to a patient.

Unfortunately, prior art devices have coupled tools and other components to a Nitinol tube in such as way as to cause stress risers and notch stresses that increase the possibility of harm to the patient. In the event such stresses cause the tube connection to break, the damage to bone and other body tissue is often catastrophic.

Although others have designed systems that seek to avoid stress risers or notch stresses, these approaches have not resulted in devices that transmit sufficient torque for many orthopedic operations.

Therefore, what is needed is a device that connects to an elastic tube in a manner that does not create stress risers. More specifically, what is needed is a reliable means of connecting various types of tool heads to an elastic tube such that significant torque may be transmitted without raising internal stresses to dangerous levels.

### Disclosure of the Invention

According to the invention, a torque-transmitting assembly is described in claim 1. Preferably, the relative motion is axial, with respect to a longitudinal axis around which torque is being transmitted through the fixed assembly.

In one or more preferred embodiments of the invention, the contact established is a surface-to-surface contact that frictionally transmits an applied torque.

In another preferred embodiment of the invention, the female coupling member may be adapted for connection with a powered driving instrument or may be a fitting adapted to present a tool-bit. Similarly, the radially flexible sleeve may be adapted for connection with a powered driving instrument or may be a fitting adapted to present a tool bit.

In yet another preferred embodiment of the invention, the radially flexible sleeve member has a plurality of collet fingers, which preferably contact the shaft at discrete locations. It is further preferred that the super-elastic activation in the shaft occurs as a result of the discrete contact thereby changing the cross-sectional shape of the shaft, which is generally non-circular, e.g., polygonal, in a further preferred form. In still another preferred embodiment of the invention, the through bore of the radially flexible sleeve member has a generally non-circular, e.g., polygonal cross-section. Alone or in combination with the immediately aforesaid feature, the shaft may be generally cylindrical and deformed, e.g., polygonally, by compression against the through-bore.

In still another preferred embodiment, the shaft has a tubular shape defining a cannulation. More preferably, the tapered bore of the female coupling member, the through-bore of the sleeve member and the cannulation of the shaft member are concentrically aligned with respect to a longitudinal axis around which torque is transmitted through the fixed assembly, further admitting passage of a guide wire through the members.

In another of its aspects, a surgical device of the present invention includes the torque transmitting coupling assembly of the invention.

In yet another of its aspects, a flexible surgical reamer of the invention is described, including the torque transmitting coupling assembly of the invention. Such a reamer is defined in claim 18.

An advantage of the present invention is a coupling assembly for an elastic tube that transmits torque without creating stress risers. More specifically, there is provided a reliable means of connecting various types of tool heads to an elastic tube such that significant torque may be transmitted without raising internal stresses to dangerous levels. Such an advantage is particularly beneficial in surgical devices that require relatively high torque-transmission, while demanding the utmost safety in operation, e.g., flexible-reaming systems.

Another advantage is the ready assembling and disassembling of the present couplings, due to the properties of the preferred nitinol alloy.

Other objects and advantages will become apparent to those skilled in the art, upon reviewing the Figures of the Drawings, in conjunction with the Detailed Description set forth further below, wherein references to numerals corresponds to like references in the Drawings.

### Brief Description of Drawings

**Fig. 1** is an exploded, sectional view of a torque-transmitting coupling according to an embodiment of the present invention, shown prior to assembly;
**Figs. 1A-C** are sequential views, partially broken away, of the assembly of **Fig. I** further being completed;
**Fig. 2** is an enlarged view of a preferred sleeve member of the present coupling as depicted in **Fig. 1;**
**Fig. 2A** is a cross sectional view taken along lines **IIA-IIA** of **Fig. 2A;**
**Fig. 3** is a longitudinal sectional view showing another embodiment of the present invention, in an assembled state;
**Fig. 3A** is a cross sectional view taken along lines **IIIA-IIIA** of **Fig. 3**, before assembly;
**Fig. 3B** is a sequential view, taken similarly along lines **IIIB-IIIB** of **Fig. 3** but after assembly, showing polygonal deformation of the tubular member according to the invention;
**Fig. 4** is a cross sectional view of yet another, alternative embodiment of the invention, taken similarly to **Figs. 3A** and **3B** after assembly, showing a cloverleaf-shaped deformation of the tubular member;
**Figs. 5-9** are sequential views showing a surgical reamer with two coupling assemblies, according to an aspect of the present invention, with a coupling located at a respective end of the tubular member;
**Fig. 9A** is a cross-section taken at lines **9A-9A** of **Fig. 9** showing reamer in an assembled state.
**Fig. 10** is an enlarged elevational view of the preferred integral tongue shown by the encircled area of **Fig. 1;**
**Fig. 10A** is an end view of **Fig. 10;**
**Fig. 10B** is an elevational view further showing the construction of the preferred integral tongue of **Figs. 10A-B;**
**Fig. 11** is an enlarged view of the encircled region **11** of **Fig. 1;**
**Fig. 12** is an enlarged view of the encircled region **12** of **Fig. 8**; and
**Fig. 13** is an enlarged exploded view of the reamer shown in **Fig. 5.**

### Brief Description of Drawings

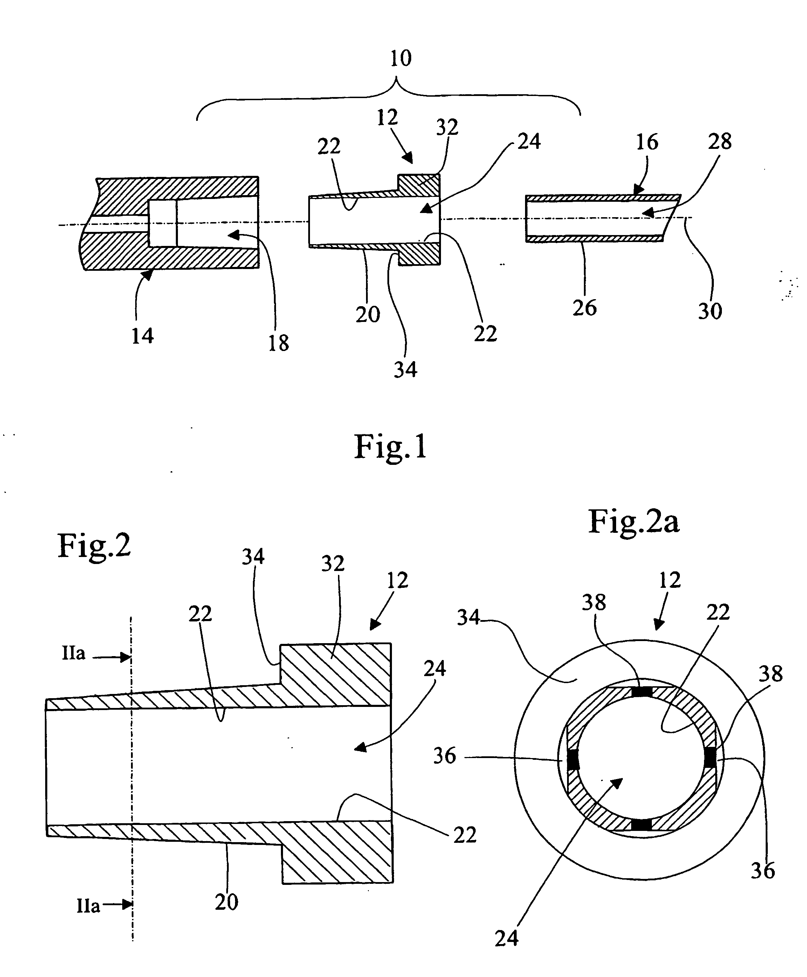

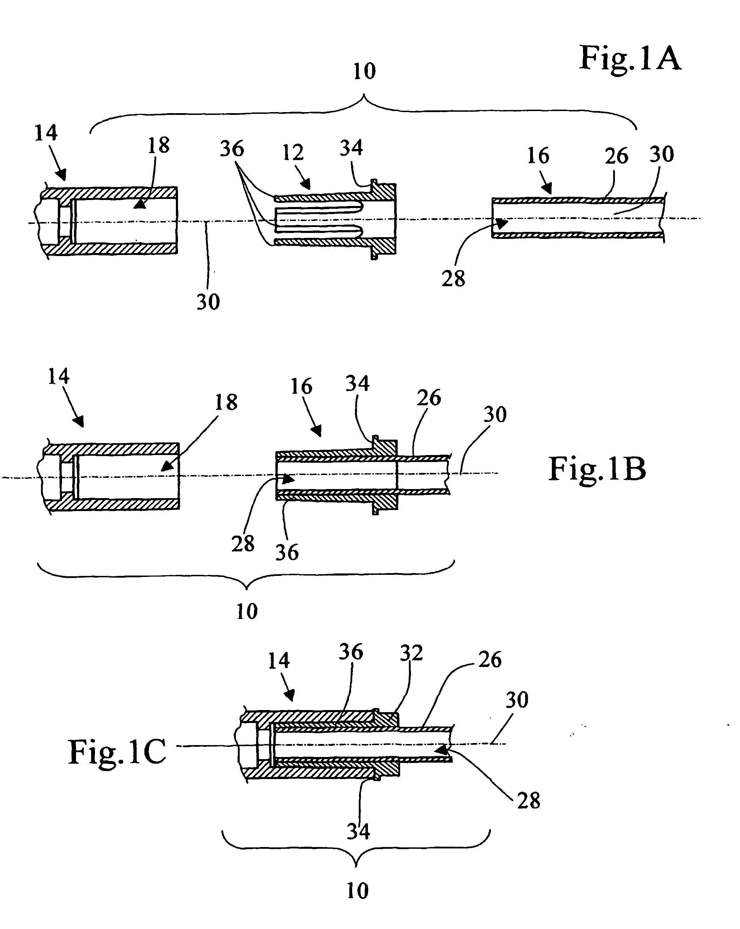

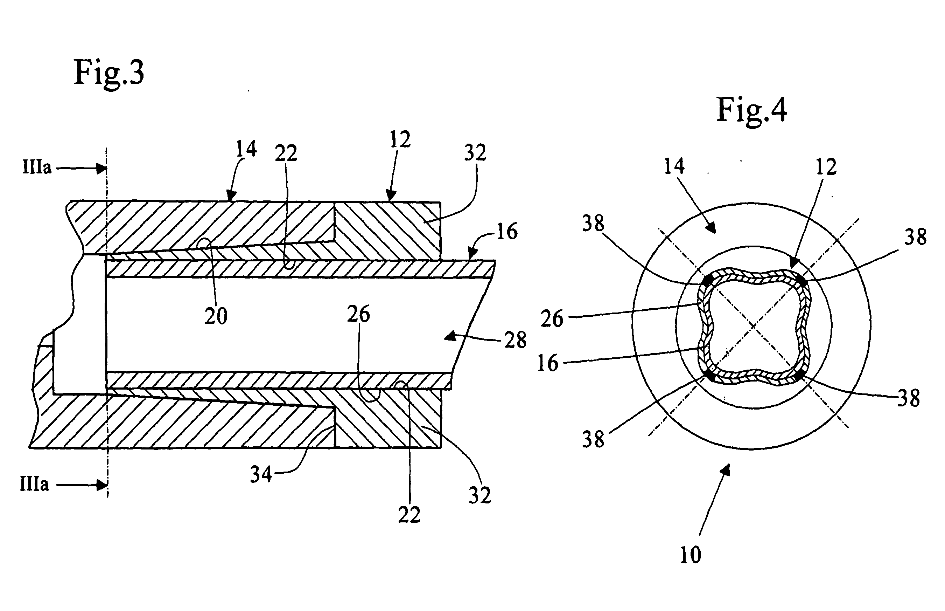

Referring to **Figs. 1-2** and **2A**, there is generally shown at **10** a preferred torque-transmitting assembly of the present invention. Assembly **10** includes a radially flexible sleeve member shown at **12,** a female coupling member shown at **14** and an elongated shaft member shown at **16**. Female coupling member **12** defines a shape with a tapered bore indicated at **18.** Sleeve **12** has a tapered exterior surface **20,** received within bore **18**, and an inner surface **22** defining a through-bore indicated at **24**. Shaft **16** is made of a super-elastic alloy, e.g., nickel-titanium (commonly known as N itinol), having an outer surface **26** received within through-bore **24** and being formed with a cannulation indicated at **28.** Relative motion among at least two of the members **12, 14, 16** causes inner surface **22** to contact outer surface **26,** inducing a super-elastic activation in shaft 16, simultaneously securing the members together in a fixed relative position. The relative motion is preferably axial, as shown for example by sequential **Figs. 1A-C,** with respect to a longitudinal axis **30** around which torque is being transmitted through the fixed assembly (**Fig. 1C**).

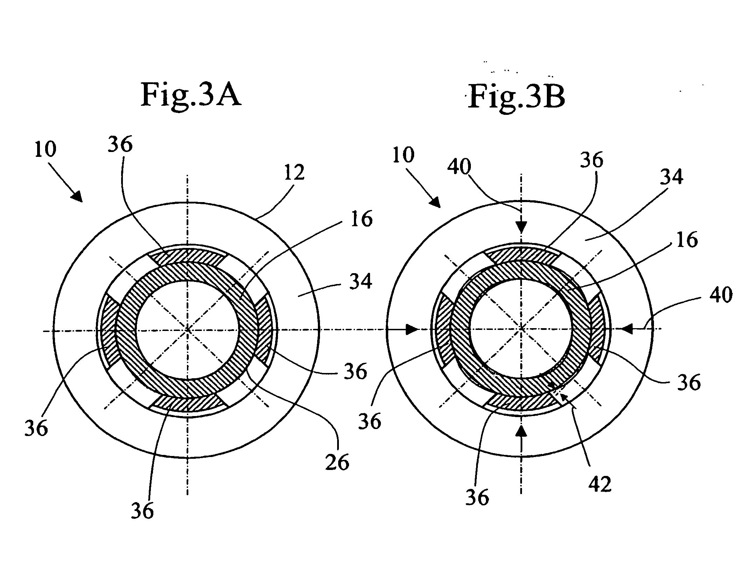

Referring to **Figs. 1A-C, 2A** and **3A-B,** the radial flexibility of sleeve **12** is imparted by its structure, which preferably includes an annular base portion **32** with shoulder **34** and a plurality of collet fingers **36** separated by splits **38** allowing flexure of the fingers. Preferably, an array of four fingers **36** are provided that exert orthogonal radial forces indicated by arrows **40** in **Fig. 3B,** resulting in a distortion of tubular shaft **16** from a cylindrical shape in **Fig. 3A** to a more polygonal shape as indicated, e.g., by the arrows **42.** As shown in **Fig. 2A,** fingers **36** may have flats **44** to minimize contact with the shaft (not shown) except where deformational forces are desired to be exerted. Alternatively, the fingers **36** could be curved as shown in **Figs. 3A-3B.** Deformation of shaft 16 restricts the sliding of the shaft in the fixed assembly **10** as it is transmitting torque.

The afore-mentioned contact may be established in a surface-to-surface manner that serves to frictionally transmit an applied torque generated about axis **30**. Nitinol has the advantage of deforming under compression to establish an area of contact, rather than a line of contact as in conventional press-fit or tapered junctions. This may be further accomplished in a selective manner by appropriate adaptation of the fingers **36** while using a standard cylindrical shaft **16** as shown in the numerous embodiments presently being depicted and suggested.

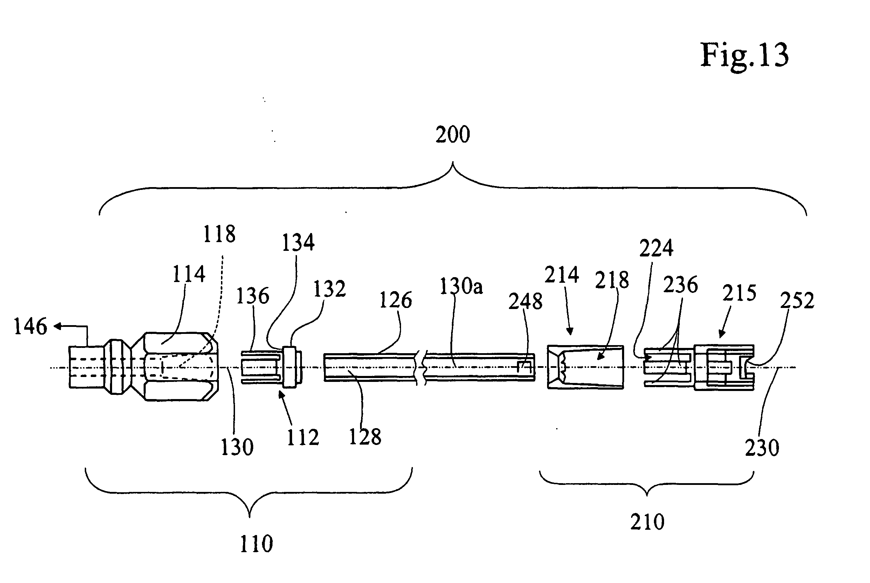

In a later described aspect of the invention embodied by a surgical reamer shown generally at **200** in **Fig. 13,** the female coupling member **114** is adapted for connection with a powered driving instrument (arrow **146**), receiving the split collet sleeve **112** to connect and drive the shaft **116** in torque-transmitting assembly **110.** Alternatively or in combination, another fitting generally indicated at **214** may be adapted to present a tool-bit generally indicated at **215** driven by shaft **116** in torque-transmitting assembly **210.**

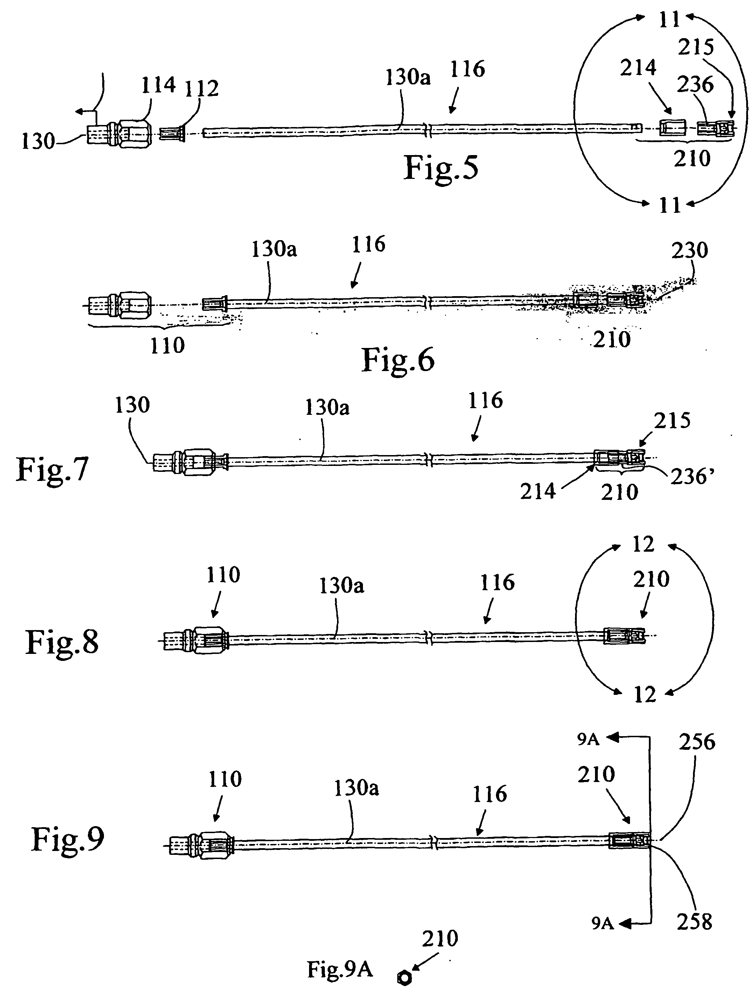

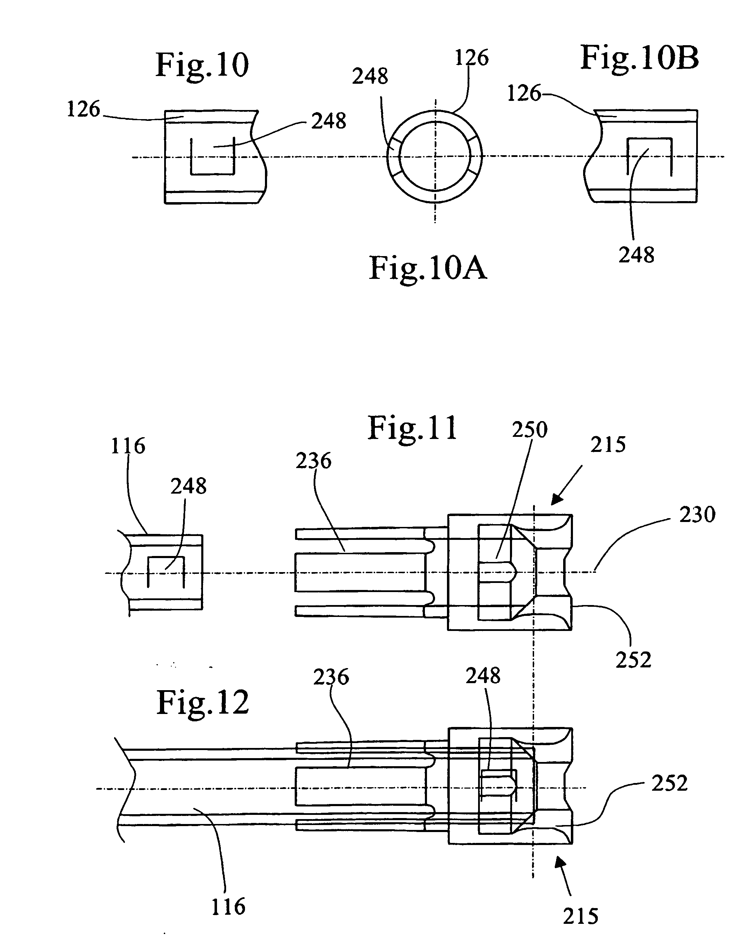

The preferred construction of tool-bit **215** is shown in **Figs. 11-13,** meanwhile, **Figs. 10A-C** depict a tongue **248** formed on shaft **116** that is received within tool-bit **215** as further shown by **Fig. 9A** in the fixed assembly **210.** Fitting **214** is positioned to allow tongue **248** to extend axially within tool bit **215 (Fig. 6).** The radially flexible sleeve **212** extends axially into fitting **214** and presents tool bit **215. Figs. 10** and **10A-B** illustrate the formation of tongue **248** in shaft **16,** which in turn allows the shaft to be detained via the tongue within a recess **250** formed in tool-bit **215 (Figs. 11-12)**.

Returning to **Figs 1-3** and associated views, the radially flexible sleeve member **12** has a plurality of collet fingers **36,** which preferably contact shaft **16** at discrete locations. It is further preferred that the super-elastic activation in shaft **16** occurs as a result of the discrete contact thereby changing the cross-sectional shape of the shaft from a circular to a generally non-circular, e.g., polygonal shape (**Figs. 3A-B**). In still another preferred embodiment of the invention, through bore **24** of radially flexible sleeve member **12** has a generally non-circular, e.g., polygonal cross-section. As a result shaft **16** is deformed, e.g., polygonally, by compression against through-bore **24.**

More preferably, tapered bore **18** of female coupling member **14,** through-bore **24** of sleeve member **12** and cannulation **28** of shaft member **16** are concentrically aligned with respect to longitudinal axis **30** around which torque is transmitted through the fixed assembly **10,** further admitting passage of a guide wire (not shown) through the members.

Referring again to **Figs. 1-3** and associated views, a torque-transmitting assembly **10** of the present invention has a female coupling member **14** defining a shape with a tapered bore **18** and has a radially flexible sleeve member **12** having a tapered exterior surface **20,** received within the bore. Sleeve **12** has an inner surface defining a through-bore **24** and has a plurality of collet fingers **36.** An elongated tubular shaft member **16** has a cannulation **28** and is made of super-elastic alloy, defining an outer surface **26** that is received within through-bore **24.** Relative axial motion among at least two of the members **12, 14,16** causes collet fingers **36** to contact the shaft at discrete locations, inducing a super-elastic activation in the shaft that changes its cross-sectional shape and simultaneously secures the members together in a fixed relative position by surface-to-surface contact that transmits torque through the assembly **10. Fig. 1A** shows shaft **16** moved axially into sleeve **12** prior to introduction within bore **18** to complete the fixed assembly **10.** Preferably, tapered bore **18** of female coupling member **14,** through-bore **24** of sleeve **12** and cannulation **28** in shaft **16** are concentrically aligned with respect to a longitudinal axis **30** around which torque is transmitted through the fixed assembly **10,** further admitting passage of a guide wire (not shown) through the members. In a preferred form, shaft **16** is generally cylindrical then is deformed polygonally by compression against through-bore **24.**

**Fig. 4** shows an alternative form of the assembly **10,** with sleeve **12** having a clover-leaf structure that imparts a corresponding deformation of shaft **16.** The sleeve is a collet with splits **38.**

In another of its aspects, a surgical device of the present invention is generally shown at **200** in **Figs. 5-13.** Device **200** includes a torque transmitting coupling assembly 110, which has a female coupling member **114** defining a shape with a tapered bore **118** and a radially flexible sleeve member **112** having a tapered exterior surface **120,** received within the bore. Sleeve **112** has an inner surface **122** defining a through-bore **124** and a plurality of collet fingers **136.** An elongated shaft member **116** made of super-elastic alloy has an outer surface **126** that is received within through-bore **124.** Relative axial motion among at least two of the members **112, 114, 116** causes inner surface **122** to engage outer surface **126,** inducing a super-elastic activation in shaft **116,** simultaneously securing the members together in a surface-to-surface contact that frictionally transmits an applied torque around a longitudinal axis **130** of the assembly **110,** securing the members together in fixed relative position. Through-bore **124** of the radially flexible member **112** preferably has a generally polygonal cross-section, as described above in relation to **Figs. 1-3.** The female coupling member **114** may be adapted for connection with a powered driving instrument (arrow **146)** or it may be a fitting **214** adapted to present a tool-bit **215.** Preferably, the tapered bore 118 of female coupling member **114,** through-bore **124** of sleeve member **112** and cannulation **128** of shaft member **116** are concentrically aligned with respect to a longitudinal axis **230** around which torque is transmitted through the fixed assembly **210,** further admitting passage of a guide wire (not shown) through the members.

In yet another of its aspects, a flexible surgical reamer of the invention is generally depicted at **200** in **Figs. 5-13.** Reamer **200** includes an elongated tubular shaft **116** made of super-elastic alloy and defining an outer surface **126** with a cannulated passageway **128** extending along a first axis **130a.** Shaft **116** is driven by a first torque-transmitting coupling assembly **110** connected to a drive source 146 and, in turn, drives another torque-transmitting assembly **210** having a tool-bit **215.** Assembly **110** includes a drive fitting **114** at its driven end, defining a shape with a first tapered bore **118,** also a radially flexible sleeve **112** having a tapered exterior surface **120,** received within the first bore **118.** Sleeve **112** has an inner surface **122** defining a through-bore **124** and a plurality of collet fingers **136**. Relative motion among at least two of the shaft **116,** fitting **114** and sleeve **112** causes through-bore **124** to contact shaft **116,** inducing a super-elastic activation in the shaft, simultaneously securing the shaft, fitting and sleeve together in fixed relative position. Reamer **200** has a second coupling assembly **210** that includes a fitting **214** defining a shape with a tapered bore **218,** a tool-bit **215** having a radially flexible portion vis-à-vis collet fingers **236** and a second axis **230,** presenting a first mating interface surface **252.** A tool (not shown herein), is of the type described in Applicant's incorporated parent application, having a tool axis **256** and presenting a second mating interface surface **258** adapted for receptive complemental facing with first mating interface surface **252,** (see Case 28NP and its prior provisional application Ser. No. 60/338/718). A deformable tongue **248** made of super-elastic alloy, is located adjacent the driving end, preferably formed from the driving end of shaft **116** (Figs. **10-14**). Relative motion between tool-bit **215** and the tool slides the first **252** and second **258** mating interface surfaces sideways into juxtaposition with the first, second and tool axes aligned with one another.

The present invention therefore provides an advantageous coupling assembly **110, 210** for an elastic tube **16, 116** that transmits torque without creating stress risers. More specifically, there is provided a reliable means of connecting various types of tool heads **215** to an elastic tube **16, 116** such that significant torque may be transmitted without raising internal stresses to dangerous levels. Such an advantage is particularly beneficial in surgical devices, such as the reamer **200** that require relatively high torque-transmission, while demanding the utmost safety in operation, e.g., flexible-reaming systems.

The non-cylindrical/non-conical forms employed by the inventive assembly **110, 210** can be created using a number of known processes. For example, these forms can be machined on a milling machine or an Electronic Discharge Milling ("EDM") machine. These forms can also be molded using investment casting and die molding techniques as well as a number of other methods.

The following parallel applications are referred is Ser. No. 10/043,423 (Attorney Case No. 25NP PREC), filed on January 10, 2002 and entitled *"Drive Shaft Coupling*", US provisional application Ser. No. 60/338,718 (Attorney Case No. 28 PREC), filed November 5, 2001 and entitled "*Tool Bit Drive Shaft Connection and Method*", as is Ser. No. 10/055,806 (Attorney Case No. 28NP PREC), filed January 22, 2002.

Multiple variations and modifications are possible in the embodiments of the invention described here. Although certain illustrative embodiments of the invention are shown and described here, a wide range of modifications, changes, and substitutions is contemplated in the foregoing disclosure. Accordingly, it is appropriate that the foregoing description be construed broadly and understood as being given by way of illustration and example only, the scope of the invention being limited only by the appended claims.

## Claims

1. A torque-transmitting assembly (10, 110, 210) comprising:
a female coupling member (14, 114, 214) and an elongated shaft member (16, 116) **characterized in that** the female coupling member (14,114, 214) defines a shape with a tapered bore (18, 118, 218), **in that** it comprises a radially flexible sleeve member (12, 112) having a wall with a tapered exterior surface (20, 120), received within the bore (18, 118), and an inner surface defining a through-bore (24, 124); **in that** the elongated shaft member (16, 116) is made of super-elastic alloy and has an outer surface (26, 126), received within the through-bore (24, 124), wherein the members are secured together in a fixed relative position
by relative motion among at least two of the members (12, 14, 16; 112, 114, 116, 214) which causes the inner surface to contact the outer surface, inducing a super-elastic activation in the shaft member (16, 116).

2. The assembly of Claim 1 wherein the contact further comprises a surface-to-surface contact that frictionally transmits an applied torque.

3. The assembly according to Claim 1 or 2 wherein the radially flexible sleeve member (12, 112) has a plurality of collet fingers (36, 136).

4. The assembly according to to Claim 3 wherein the collet fingers (36, 136) contact the shaft member (16, 116) at discrete locations.

5. The assembly according to Claim 4 wherein the super-elastic activation in the shaft member (16, 116) occurs as a result of the discrete contact thereby changing the cross-sectional shape of the shaft.

6. The assembly according to Claim 5 wherein the resultant cross-sectional shape of the shaft member (16, 116) is generally non-circular.

7. The assembly of Claim 6 wherein the resultant shape of the shaft member (16, 116) is generally polygonal.

8. The assembly according to one of the Claims 1 to 7 wherein the through bore (24, 124) of the radially flexible sleeve member (12, 112) has a generally non-circular cross-section.

9. The assembly according to Claim 8 wherein the through bore (24, 124) has a generally polygonal cross-section.

10. The assembly according to one of the Claims 1 to 9 wherein the female coupling member (14, 114, 214) is either adapted for connection (14, 114) with a powered driving instrument (146)or is a fitting (214) adapted to present a tool-bit (215).

11. The assembly according to one of the Claims 1 to 9 wherein the radially flexible sleeve (12, 112) is either adapted for connection with a powered driving instrument (146) or comprises a fitting (214) adapted to present a tool bit.

12. The assembly according to one of the Claims 1 to 6 or 8 to 11 wherein the shaft member (16, 116) is generally cylindrical and is deformed polygonally by compression against the through-bore (24, 124).

13. The assembly according to one of the Claims 1 to 12 wherein the relative motion is axial, with respect to a longitudinal axis (30, 130,230)) around which torque is being transmitted through the fixed assembly.

14. The assembly according to one of the Claims 1 to 13 wherein the shaft member (16, 116) has a tubular shape defining a cannulation (128).

15. The assembly according to Claim 14 wherein the tapered bore (18, 118, 218) of the female coupling member, the through-bore (24, 124) of the sleeve member (12, 112) and the cannulation (128)of the shaft member are concentrically aligned with respect to a longitudinal axis (30, 130, 230) around which torque is transmitted through the fixed assembly, further admitting passage of a guide wire there-through.

16. The assembly according to one of the Claims 1 to 15 wherein the super-elastic alloy is a nickel-titanium alloy.

17. A surgical device (200) including a torque transmitting coupling assembly (110) according to one of the claims 1 to 16.

18. A flexible surgical reamer (200) comprising a first torque transmitting assembly (110) according to claim 1 **characterized in that**
a) the elongated tubular shaft member (116) defines a passageway (128)extending along a first axis (130a), with a driven end and a driving end opposite the driven end;
b) the first torque transmitting assembly(110) includes
(i) a drive fitting (146) located at the driven end, defining a shape with a first tapered bore (118),
(ii) the radially flexible sleeve member (112)has a plurality of collet fingers (136), and
c) a second torque transmitting assembly (210) that includes
(i) a fitting (214) defining a shape with a second tapered bore (218),
(ii) a tool-bit having (215) a second axis and a radially flexible sleeve portion (236) received in the second bore (218), the tool-bit presenting a first mating interface surface (252)
(iii) a tool having a tool axis (256)and presenting a second mating interface surface (258) and
(iv) a deformable tongue (248) made of super-elastic alloy, located adjacent the driving end, for detentively retaining the tool-bit (215) on the shaft member (116)
(v) whereupon relative motion between the tool-bit (215) and tool slides the first and second mating interface surfaces (252, 258) sideways into juxtaposition with the first, second and tool axes aligned with one another.

## Patentansprüche

1. Drehmomentübertragende Anordnung (10, 110, 210), enthaltend:
ein aufnehmendes Kupplungselement (14, 114, 214) und ein gestrecktes Schaftelement (16, 116),
**dadurch gekennzeichnet, dass** das aufnehmende Kupplungselement (14, 114, 214) eine Form mit einer sich verjüngenden Bohrung (18, 118, 218) aufweist, dass die Anordnung eine radial nachgiebige Hülse (12, 112) enthält, deren Wandung eine sich verjüngende Aussenfläche (20, 120) besitzt, welche in der Bohrung (18, 118) aufgenommen werden kann, sowie eine Innenfläche, die eine Durchgangsbohrung (24, 124) darstellt; dass das gestreckte Schaftelement (16, 116) aus einer superelastischen Legierung hergestellt ist und eine Aussenfläche (26, 126) aufweist, die von der Durchgangsbohrung (24, 124) aufgenommen wird,
wobei die Bauteile in einer gegenseitig festgelegten Stellung durch eine gegenseitige Bewegung von mindestens zwei der Bauteile (12, 14, 16; 112, 114, 116, 214) gesichert werden, wodurch die Innenfläche in Berührung mit der Aussenfläche kommt sowie eine superelastische Aktivierung im Schaftelement herbeigeführt wird.

2. Anordnung nach Anspruch 1, bei welcher der Kontakt weiterhin einen Kontakt von Oberfläche zu Oberfläche umfasst, der ein angelegtes Drehmoment reibungsschlüssig überträgt.

3. Anordnung nach Anspruch 1 oder 2, bei der die radial nachgiebige Hülse (12, 112) mehrere Spannfinger (36, 136) aufweist.

4. Anordnung nach Anspruch 3, bei der die Spannfinger (36, 136) das Schaftelement (16, 116) an voneinander getrennten Stellen berühren.

5. Anordnung nach Anspruch 4, bei der die superelastische Aktivierung im Schaftelement (16, 116) als Ergebnis der einzelnen Berührungsstellen auftritt, wobei die Querschnittsform des Schaftes verändert wird.

6. Anordnung nach Anspruch 5, bei der sich ergebende Querschnittsform des Schaftelementes (16, 116) allgemein nicht kreisförmig ist.

7. Anordnung nach Anspruch 6, bei der die sich ergebende Form des Schaftelementes (16, 116) allgemein vieleckig ist.

8. Anordnung nach einem der Ansprüche 1 bis 7, bei der die Durchgangsbohrung (24, 124) des radial nachgiebigen Schaftelementes (12, 112) allgemein einen nicht kreisförmigen Querschnitt aufweist.

9. Anordnung nach Anspruch 8, bei der die Durchgangsbohrung (24, 124) eine allgemein vieleckige Querschnittsform aufweist.

10. Anordnung nach einem der Ansprüche 1 bis 9, bei der das aufnehmende Kupplungselement (14, 114, 214) entweder zur Verbindung (14, 114) mit einem motorischen Antriebsinstrument (146) eingerichtet ist oder ein Anschlussstück (214) ist, welches zur Aufnahme eines Werkzeugeinsatzes (215) eingerichtet ist.

11. Anordnung nach einem der Ansprüche 1 bis 9, bei der die radial nachgiebige Hülse (12, 112) entweder zur Verbindung mit einem motorischen Antriebsinstrument (146) eingerichtet ist oder ein Anschlussstück (214) aufweist, das zur Aufnahme eines Werkzeugeinsatzes eingerichtet ist.

12. Anordnung nach einem der Ansprüche 1 bis 6 oder 8 bis 11, bei der das Schaftelement (16, 116) allgemein zylindrisch ist und durch Kompression gegen die Durchgangsbohrung (24, 124) vieleckig verformt wird.

13. Anordnung nach einem der Ansprüche 1 bis 12, bei der die Relativbewegung bezüglich einer Längsachse (30, 130, 230) axial gerichtet ist, um welche ein Drehmoment durch die zusammengebaute Anordnung übertragen wird.

14. Anordnung nach einem der Ansprüche 1 bis 13, bei der das Schaftelement (16, 116) eine rohrförmige Gestalt besitzt, welche einen Durchgangskanal (128) definiert.

15. Anordnung nach Anspruch 14, bei der die sich verjüngende Bohrung (18, 118, 218) des aufnehmenden Kupplungselementes, die Durchgangsbohrung (24, 124) der Hülse (12, 112) und der Durchgangskanal (128) des Schaftelementes konzentrisch auf eine Längsachse (30, 130, 230) ausgerichtet sind, um welche durch die befestigte Anordnung ein Drehmoment übertragen wird, wobei zusätzlich der Durchgang eines Führungsdrahtes durch die Anordnung ermöglicht wird.

16. Anordnung nach einem der Ansprüche 1 bis 15, bei der die superelastische Legierung eine Nickel-Titan-Legierung ist.

17. Chirurgische Vorrichtung (200), enthaltend eine drehmomentübertragende Kupplungsanordnung (110) nach einem der Ansprüche 1 bis 16.

18. Elastische chirurgische Reibahle (200), mit einer ersten drehmomentübertragenden Anordnung (110) nach Anspruch 1, **dadurch gekennzeichnet, dass**
(a) das gestreckte rohrförmige Schaftelement (116) einen Durchgang (128) definiert, der sich entlang einer ersten Achse (130a) erstreckt, mit einem Antriebsende und einem dem Antriebsende entgegengesetzten Abtriebsende;
(b) die erste drehmomentübertragenden Anordnung (110)
(i) einen Antriebanschluss (146), aufweist, der sich am Antriebsende befindet und eine sich verjüngende Bohrung (118) enthält, wobei
(ii) die radial nachgiebige Hülse (112) mehrere Spannfinger (136) aufweist, und
(c) eine zweite drehmomentübertragende Anordnung (210), die
(i) ein Anschlussstück (214) mit einer zweiten sich verjüngenden Bohrung (218),
(ii) einen Werkzeugeinsatz (215) mit einer zweiten Achse und einem radial nachgiebigen Hülsenbereich (236), der in der zweiten Bohrung (218) aufgenommen wird, wobei der Werkzeugeinsatz eine erste Verbindungs-Passfläche (252) besitzt,
(iii) ein Werkzeug mit einer Werkzeugachse (256) und mit einer zweiten Verbindungs-Passfläche (258), und
(iv) eine verformbare Zunge (248) aus einer superelastischen Legierung, die benachbart zum Abtriebsende angeordnet ist, zwecks lösbarer Befestigung des Werkzeugeinsatzes (215) am Schaftelement (116)
(v) aufweist, wobei eine Relativbewegung zwischen dem Werkzeugeinsatz (215) und dem Werkzeug die beiden Verbindungs-Passflächen (252, 258) seitwärts in Nebeneinaderstellung mit der ersten und zweiten Achse und mit der Werkzeugachse bringt, welche aufeinander ausgerichtet sind.

## Revendications

1. Assemblage pour transmission de couple (10, 110, 210), comprenant
un élément d'accouplement femelle (14, 114, 214) et une tige allongée (16, 116),
**caractérisé en ce que** l'élément d'accouplement femelle (14, 114, 214) définit une forme avec un alésage conique (18, 118, 218), **en ce que** l'assemblage comprend une douille radialement flexible (12, 112) dont la paroi présente une surface extérieure conique (20, 120) reçue dans l'alésage (18, 118), et une surface intérieure définissant un alésage traversant (24, 124), **en ce que** la tige allongée (16, 116) est fabriquée à partir d'un alliage super-élastique et présente une surface extérieure (26, 126) reçue dans l'alésage traversant (24, 124);
dans lequel les éléments sont attachés les uns aux autres dans une position relative fixe par un mouvement relatif entre au moins deux des éléments (12, 14, 16; 112, 114, 116, 214) ce qui amène la surface intérieure en contact avec la surface extérieure en induisant une activation super-élastique dans la tige (16, 116).

2. Assemblage selon la revendication 1, dans lequel le contact comprend en plus un contact de surface à surface qui transmet par friction un couple appliqué.

3. Assemblage selon la revendication 1 ou 2, dans lequel la tige radialement flexible (12, 112) comporte une pluralité de doigts de serrage (36, 136).

4. Assemblage selon la revendication 3, dans lequel les doigts de serrage (36, 136) sont en contact avec la tige (16, 116) à des endroits discrets.

5. Assemblage selon la revendication 4, dans lequel l'activation super-élastique dans la tige (16, 116) se produit comme résultat du contact discret accompagné d'un changement de la forme de la section transversale de la tige.

6. Assemblage selon la revendication 5, dans lequel la forme résultante de la section transversale de la tige (16, 116) est généralement non circulaire.

7. Assemblage selon la revendication 6, dans lequel la forme résultante de la tige (16, 116) est généralement polygonale.

8. Assemblage selon l'une des revendications 1 à 7, dans lequel l'alésage traversant (24, 124) de la tige radialement flexible (12, 112) présente une section transversale généralement non circulaire.

9. Assemblage selon la revendication 8, dans lequel l'alésage traversant (24, 124) présente une section transversale généralement polygonale.

10. Assemblage selon l'une des revendications 1 à 9, dans lequel l'élément d'accouplement femelle (14, 114, 214) est soit adapté à une connexion (14, 114) avec un instrument d'entraînement actionné par moteur (146), soit un raccord (214) adapté pour présenter une partie d'outil (215).

11. Assemblage selon l'une des revendications 1 à 9, dans lequel la tige radialement flexible (12, 112) est soit adaptée à une connexion avec un instrument d'entraînement actionné par moteur (146), soit comprend un raccord (214) adapté pour présenter une partie d'outil.

12. Assemblage selon l'une des revendications 1 à 6 ou 8 à 11, dans lequel la tige (16, 116) est généralement cylindrique et est déformée de manière polygonale par une compression contre l'alésage traversant (24, 124).

13. Assemblage selon l'une des revendications 1 à 12,
dans lequel le mouvement relatif est axial par rapport à un axe longitudinal (30, 130, 230) autour duquel un couple est transmis par l'intermédiaire de l'assemblage fixé.

14. Assemblage selon l'une des revendications 1 à 13,
dans lequel la tige (16, 116) présente une forme tubulaire définissant un conduit tubulaire (128).

15. Assemblage selon la revendication 14, dans lequel l'alésage conique (18, 118, 218) de l'élément d'accouplement femelle, l'alésage traversant (24, 124) de la douille (12, 112), et le conduit tubulaire (128) de la tige sont alignés de façon concentrique par rapport à un axe longitudinal (30, 130, 230) autour duquel un couple est transmis par l'intermédiaire de l'assemblage fixé, permettant en outre le passage traversant d'un fil de guidage.

16. Assemblage selon l'une des revendications 1 à 15,
dans lequel l'alliage super-élastique est un alliage de nickel et de titane.

17. Dispositif chirurgical (200) comprenant un assemblage d'accouplement pour transmission de couple (110) selon l'une des revendications 1 à 16.

18. Alésoir chirurgical souple (200) comprenant un premier assemblage pour transmission de couple (110) selon la revendication 1, **caractérisé en ce que**
a) la tige tubulaire allongée (116) définit une voie de passage (128) s'étendant le long d'un premier axe (130a) avec une extrémité entraînée et une extrémité d'entraînement opposée à l'extrémité entraînée;
b) le premier assemblage pour transmission de couple (110) comprend
i) un raccord d'entraînement (146) situé à l'extrémité entraînée et présentant un premier alésage conique (118),
ii) la douille radialement flexible (112) comportant une pluralité de doigts de serrage (136), et
c) un deuxième assemblage pour transmission de couple (210) qui comprend
i) un raccord (214) définissant une forme avec un deuxième alésage conique (218),
ii) une partie d'outil (215) ayant un deuxième axe et une partie de douille radialement flexible (236) reçue dans le deuxième alésage (218), la partie d'outil présentant une première surface de jonction appariée (252),
iii) un outil ayant un axe d'outil (256) et présentant une deuxième surface de jonction appariée (258), et
iv) une languette déformable (248) faite en un alliage super-élastique, située à proximité de l'extrémité d'entraînement, afin de retenir la partie d'outil (215) sur la tige (116),
v) où un mouvement relatif entre la partie d'outil (215) et l'outil fait glisser latéralement les première et deuxième surfaces de jonction appariées (252, 258) dans une position juxtaposée, le premier, le deuxième et les axes de l'outil étant alignés les uns aux autres.
